# EUROPEAN PATENT SPECIFICATION

(11) **EP 4 378 448 B1**
(45) Date of publication and mention of the grant of the patent: **20.05.2026**
(21) Application number: 22214701.9
(22) Date of filing: 19.12.2022
(51) Int. Cl.: A61K 8/9789, A61K 8/9794, A61Q 19/02, A61Q 19/08, A23L 33/105

(54) **EXTRACT OF FLOWERS, LEAVES AND/OR SEED PODS OF IMPATIENS AND LEAVES OF PANDANUS, IN DRIED AND LIQUID FORM, A METHOD OF ITS OBTAINING AND USE THEREOF**
EXTRAKT AUS BLÜTEN, BLÄTTERN UND/ODER SAMENSCHALEN VON SPRINGKRAUT UND BLÄTTERN VON PANDANUS IN GETROCKNETER UND FLÜSSIGER FORM, VERFAHREN ZU DESSEN GEWINNUNG UND VERWENDUNG
EXTRAIT DE FLEURS, FEUILLES ET/OU CAPSULES DE SEMENCES D'IMPATIENTE ET DE FEUILLES DE PANDANUS, SOUS FORME SÉCHÉE ET LIQUIDE, SON PROCÉDÉ D'OBTENTION ET SON UTILISATION

(30) Priority: 30.11.2022 PL 44300422
(43) Date of publication of application: 05.06.2024
(73) Proprietor: PBCLABS Sp. z o.o., 20-150 Lublin (PL)
(72) Inventor: DOS SANTOS SZEWCZYK, Katarzyna, 21-003 Dys (PL)
(74) Representative: Wlasienko, Jozef

(56) References cited:
- WO-A1-2022/004669
- JP-A- 2003 342 153
- JP-A- 2015 232 072
- KUMAR PRAMOD ET AL: "Comparative study on conventional, ultrasonication and microwave assisted extraction of [gamma]-oryzanol from rice bran", JOURNAL OF FOOD SCIENCE AND TECHNOLOGY, SPRINGER (INDIA) PRIVATE LTD, INDIA, vol. 53, no. 4, 16 April 2016 (2016-04-16), pages 2047 - 2053, XP035986410, ISSN: 0022-1155, [retrieved on 20160416], DOI: 10.1007/S13197-016-2175-2

## Description

### Technical Field

The subject of the invention is an extract from flowers, leaves and/or seed pods of Impatiens and leaves of Pandanus, in dry and liquid form, a method of its obtaining and use threof. In particular, the invention includes an extract obtained from *Impatiens glandulifera* with the addition of *Pandanus amaryllifolius,* characterized by a high total polyphenol content and having application in cosmetics and dietary supplements.

### Background

The Impatiens L. genus (F. Balsaminaceae) comprises nearly 500 species (http://www.worldfloraonline.org), of which the following occur in the Polish flora: *Impatiens noli - tangere, I. glandulifera* Royle, *I. parviflora* and *I. capensis.* The species native to the area of Poland is *I. noli-tangere,* while the other three are established anthropophytes that are part of the vascular flora of the area of Poland (Mirek, Z., Pi 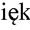 ko-ś-Mirkowa, H., Zaj 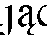 c, A., Zaj 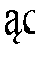 c, M. Flowering Plants and Pteridophytes of Poland. A checklist. A critical list of vascular plants of Poland. W. Szafer Institute of Botany, Polish Academy of Sciences, Cracow 2002; Romański, M. Obce gatunki - niecierpek gruczolowaty. Kwartalnik Wigry, 2012, 2, 5-6, Wigierski Park Narodowy, Krzywe).

*I. balsamina* is the most frequently described taxon in the literature. The seeds of this species were used in India as an expectorant, an antidote in fish toxin poisoning, and in women to induce menstruation and suppress postpartum pain, while the flowers were helpful in the treatment of skin burns and wounds (Kumar, B., Vijayakumar, M., Govindarajan, R., Pushpangadan, P. Ethnopharmacological approaches to wound healing - Exploring medicinal plants of India. J. Ethnopharmacol. 2007, 114, 103-113; Shoji, N., Umeyama, A., Saitou, N., Yoshikawa, K., Nagai, M., Arihara, S. Hosenkosides, F, G, H, I, J, and K, Novel baccharane glycosides from the seeds of Impatiens balsamina. Chem. Pharm. Bull. 1994, 42, 1422-1426; Shoji, N., Umeyama, A., Yoshikawa, K., Nagai, M., Arihara, S. Baccharane glycosides from seeds of Impatiens balsamina. Phytochem. 1994, 37, 1437-1441). The leaves of *I. balsamina* have been used in Thailand for fungal infections and skin ulcers, and in Korea for the treatment of boils and dysentery (Lim, Y.H., Kim, I.H., Seo, J.J. In vitro activity of kaempferol isolated from the Impatiens balsamina alone and in combination with erythromycin or clindamycin against Propionibacterium acnes. J. Microbiol. 2007, 45, 473-7; Panichayupakaranant, P., Noguchi, H., De-Eknamkul, W. A new biscoumarin from Impatiens balsamina root cultures. Planta Med. 1998, 64, 774-775.). Traditional Chinese Medicine uses *I. balsamina* for sore throats, swellings, bruises and in rheumatism (Ding, Z.S., Jiang, F.S., Chen, N.P., Lv, G.Y., Zhu, C.G. Isolation and identification of an antitumor component from leaves of Impatiens balsamina. Molecules. 2008, 13, 220-9; Yang, X., Summerhurst, K.D., Koval, S.F., Ficker, C., Smith, M.L., Bernards, M.A. Isolation of an antimicrobial compound from Impatiens balsamina L. using bioassay-guided fractionation. Phytother. Res. 2001, 15, 676-680.). Furthermore, the squeezed juice, with anti-inflammatory and anti-pruritic properties, was applied in the form of compresses on the affected skin with accompanying itching, for which naphthoquinones, as well as ferulic acid, kaempferol, quercetin and their glycosides are responsible (Ishiguro, K., Ohira, Y., Oku, H. Antipruritic dinaphthofuran-7,12-dione derivatives from the pericarp of Impatiens balsamina. J. Nat. Prod. 1998, 61, 1126-9; Ishiguro, K., Oku, H. Antipruritic effect of flavonol and 1,4-naphthoquinone derivatives from Impatiens balsamina L. Phytother. Res. 1997, 11, 343-7; Oku, H., Kato, T., Ishiguro, K. Antipruritic effects of 1,4-naphthoquinones and related compounds. Biol. Pharm. Bull. 2002, 25, 137-9; Wang, Y.C., Li, W.Y., Wu, D.C., Wang, J.J., Wu, C.H., Liao, J.J., Lin, C.K. In vitro activity of 2-methoxy-1,4-naphthoquinone and stigmasta-7,22-diene-3β-ol from Impatiens balsamina L. against multiple antibiotic-resistant Helicobacter pylori. Evid. Based Complement. Alternat. Med. 2011: 704721. doi: 10.1093/ecam/nep147.).

Studies using mice confirmed that the active compounds contained in *I. balsamina* can be used in the prevention and treatment of chronic skin inflammation, including atopy (Oku, H., Ishiguro, K. Cyclooxygenase-2 inhibitory 1,4-naphthoquinones from Impatiens balsamina L. Biol. Pharm. Bull. 2002, 25, 658-660.). The herb of this species was used to treat skin and systemic infections caused by microorganisms (Au, D.T., Wu, J., Jiang, Z., Chen, H., Lu, G., Zhao, Z. Ethnobotanical study of medicinal plants used by Hakka in Guangdong, China. J. Ethnopharmacol. 2008, 117, 41-50; Yang, X., Summerhurst, K.D., Koval, S.F., Ficker, C., Smith, M.L., Bernards, M.A. Isolation of an antimicrobial compound from Impatiens balsamina L. using bioassay-guided fractionation. Phytother. Res. 2001, 15, 676-680.). It was also found that flavonoids such as kaempferol and quercetin isolated from its flowers enhance the antibacterial effect of clindamycin and erythromycin against antibiotic-resistant strains of *Cutibacterium acnes,* one of the main causes of acne (Lim, Y.H., Kim, I.H., Seo, J.J. In vitro activity of kaempferol isolated from the Impatiens balsamina alone and in combination with erythromycin or clindamycin against Propionibacterium acnes. J. Microbiol. 2007, 45, 473-7).

Valued medicinal raw materials are also *I. textori* Miq. used for detoxification, treatment of skin lesions, injuries and boils (Tatsuzawa, F., Saito, N., Mikanagi, Y., Shinoda, K., Toki, K., Shigihara, A., Honda, T. An unusual acylated malvidin 3-glucoside from flowers of Impatiens textori Miq. (Balsaminaceae). Phytochem. 2009, 70, 672-674; Ueda, Y., Oku, H., Iinuma, M., Ishiguro, K. Effects on blood pressure decrease in response to PAF of Impatiens textori MIQ. Biol. Pharm. Bull. 2003, 26, 1505-1507), *I. siculifer* Hook.f. used in the treatment of burns, bruises, abrasions and in the treatment of rheumatoid pains (Li, W., Bi, X., Wang, K., Li, D., Satou, T., Koike, K. Triterpenoid saponins from Impatiens siculifer. Phytochem. 2009, 70, 816- 821), as well as the rhizome of *I. pritzelii* Hook.f. used in the treatment of diarrhea and acute abdominal pains (Zhou, X. F., Zhao, X. Y., Tang, L., Ruan, H. L., Zhang, Y. H., Pi, H. F., Xiao, W. L., Sun, H. D., Wu, J. Z. Three new triterpenoid saponins from the rhizomes of Impatiens pritzellii var. hupehensis. J. Asian Nat. Prod. Res. 2007, 9, 379-385). Furthermore, extracts from *I. bicolor* show antibacterial and antifungal activity (Hasan, A., Tahir, N. Flavonoids from the leaves of Impatiens bicolor. Turk. J. Chem. 2005, 29, 65-70), while ferulic acid, isolated from this species, has an inhibitory effect on the activity of acetylcholinesterase and free radicals (Shahwar, D., Rehman, S.U., Raza, M.A. Acetyl cholinesterase inhibition potential and antioxidant activities of ferulic acid isolated from Impatiens bicolor Linn. J. Med. Plants Res. 2010, 4, 260-266). *I. pallida* ointment is used in the treatment of hemorrhoids, and the juice is used to combat verrucae, warts and ringworm (Brown, D. Wielka encyklopedia zió 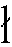 . Muza, Warszawa, 1999; Crellin, J.K., Philpott, J. A reference guide to medicinal plants: herbal medicine past and present. Duke University Press, Durham and London, 1990; Lipton, R.A. The use of Impatiens biflora (Jewelweed) in the treatment of rhus dermatitis. Ann. Allergy. 1958, 16, 526-7).

*Impatiens glandulifera* has been used in Dr. Bach's flower essences. The flower extract has a calming effect and is recommended for people who are nervous, irritated, living in constant stress (Pintov, S., Hochman, M., Livne, A., Heyman, E., Lahat, E. Bach flower remedies used for attention deficit hyperactivity disorder in children - a prospective double blind controlled study. Eur. J. Paediatr. Neurol. 2005, 9, 395-398; Vennells, D.F. Bach flowers remedies for beginners: 38 essences that heal from deep within. Llewellyn Publications, 2001). In northern India, the plant was used as a remedy against the effects of snake bites, as well as an agent restoring a state of mental relaxation (Kumar, M.; Paul, Y.; Anand, V. K. An ethnobotanical study of medicinal plants used by the locals in Kishtwar, Jammu and Kashmir, India. Ethnobotanical Leaflets. 2009, 10, 1240-1256). Recent studies indicate a cytostatic effect against U373 glioma cells of two glucosylated steroids isolated from *Impatiens glandulifera* stems (Cimmino, A., Mathieu, V., Evidente, M., Ferderin, M., Banuls, L.M.Y., Masi, M., De Carvalho, A., Kiss, R., Evidente, A. Glanduliferins A and B, two new glucosylated steroids from Impatiens glandulifera, with in vitro growth inhibitory activity in human cancer cells. Fitoterapia. 2016, 109, 138-145). The *I. noli-tangere* herb, in the form of infusions, decoctions and macerates, has antiseptic, bactericidal, diuretic, antiseptic, anti-inflammatory, astringent and coating properties on mucous membranes. Externally, it helps treat haemorrhoids, infections of the female reproductive system, and also ulcers (Fijalkowski, D., Chojnacka-Fijalkowska, E. Rośliny lecznicze na Lubelszczyźnie. Lubelskie Towarzystwo Naukowe, Lublin, 2009; Hatcher, P.E. Impatiens noli-tengere L. Biological flora of the British Isles. J. Ecol. 2003, 91, 147-167.). In the folk medicine of the Low Beskids (PL), *I. noli-tangere* was used to treat fright, a disease whose main symptoms are: restlessness during sleep, irritability, weakness, depression or headache and dizziness (Kujawska, M. (2013). Treating folk illnesses with plants by the Polish community in Misiones, Argentina. Etnobiologia Polska. 3, 31-46). In contrast, *I. parviflora* has, among others, an inhibitory effect on hyaluronidase and has the ability to counteract heat-induced denaturation of bovine serum albumin (Grabowska, K., Podolak, I., Galanty, A., Żmudzki, P., Koczurkiewicz, P., Piska, K., P 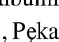 kala, E., Janeczko, Z. Two new triterpenoid saponins from the leaves of *Impatiens parviflora* DC. and their cytotoxic activity. Ind. Crops Prod. 2017, 96, 71-79).

*Pandanus amaryllifolius* Roxb. (F. Pandanaceae) comprises about 750 species (http://www.worldfloraonline.org). Extracts, mainly from the leaves and fruits of various Pandanus species, are used in traditional medicine in South and Southeast Asia and the Pacific islands in the treatment of many diseases, including digestive, respiratory and urinary tract disorders (Hoa, L.T.P.; Nhu, C.T. Phytochemical constituents and antioxidant, antimcrobial and tyrosinase inhibitory activities of Pandanus tectorius extracts. Tap. Chi, Sinh. Hoc. 2015, 37, 45-53; Ordas, J.A.D.; Nonato, M.G.; Moran, C.B. Ethnobotanical uses of Pandanaceae species in selected rural communities in the Philippines. Econ. Bot. 2021, 74, 411-428). In the Philippines, *P. amaryllifolius* is used as an ingredient of supplements supporting the body detoxification, as well as for digestive, genitourinary and circulatory problems (Ordas, J.A.D.; Nonato, M.G.; Moran, C.B. Ethnobotanical uses of Pandanaceae species in selected rural communities in the Philippines. Econ. Bot. 2021, 74, 411-428). Extracts from *P. tectorius,* especially the fruit extracts, show good antioxidant activity by scavenging DPPH radicals in correlation with their high polyphenol content. Furthermore, the extracts show strong antimicrobial activity against Gram-positive and Gram-negative bacteria, tyrosinase inhibition, as well as photoprotection (Hoa, L.T.P.; Nhu, C.T. Phytochemical constituents and antioxidant, antimcrobial and tyrosinase inhibitory activities of Pandanus tectorius extracts. Tap. Chi, Sinh. Hoc. 2015, 37, 45-53). It has been shown that the ethanol extract of *P. amaryllifolius* leaves can be used in cosmetics, such as hair preparations or cream peeling preparations. A cream scrub with 3% of the extract moisturizes the skin by 90.72% and improves the skin smoothness by 31.48% (Leny, L.; Vivi, E.D.; Ginting, M.; Iskandar, B.; Fadhila, C. The effectiveness of pandan wangi leaves (Pandanus amaryllifolius Roxb.) body scrub formulation in smoothing the skin. Asian J. Pharm. Res. Dev. 2022, 10, 1-5. doi: https://doi.org/10.22270/ajprd.v10i1.1072).

For the biological activity of *I. glandulifera* and *P. amaryllifolius* are responsible the active compounds present in them, especially the high amounts of polyphenols. The aerial parts of *I. glandulifera* contained 16.34, in leaves - 22.84, in flowers - 24.72, and in roots - 13.26 mg GAE/g DW (GAE - gallic acid equivalent, DW - dry weight) of total polyphenol content. Moreover, in the aerial parts - 3.10 mg CAE/g DW of total phenolic acid content, in leaves - 3.84 mg CAE/g DW, in flowers - 4.39 mg CAE/g DW, and in roots - 1.38 mg CAE/g DW (CAE - caffeic acid equivalent) were found. In these species also a high total flavonoid content - 5.34 mg QE/g DW in the aerial parts, 10.06 mg QE/g DW in leaves, 9.74 mg QE/g DW in flowers and 3.27 mg QE/ g DW in roots (QE - quercetin equivalent) was found (Szewczyk, K.; Zidorn, C.; Biernasiuk, A.; Komsta, L.; Granica, S. Polyphenols from Impatiens (Balsaminaceae) and their antioxidant and antimicrobial activities. Ind. Crop. Prod. 2016, 86, 262-272; Szewczyk, K.; Orzelska-Górka, J.; Polakowska, M.; Bia 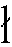 a, G. Antinociceptive and antianxiety activity of hydroalcoholic extracts of three *Impatiens* species in mice. Acta Pol. Pharm. 2018, 75, 989-1001). The leaves of *P. amaryllifolius* contained 1.12-1.87 mg QE/g DW of total flavonoid content expressed as quercetin equivalent, 4.88-6.72 mg CAE/g DW of total phenolic acid content (Ghasemzadeh, A.; Jaafar, H.Z. Profiling of phenolic compounds and their antioxidant and anticancer activities in pandan (Pandanus amaryllifolius Roxb.) extracts from different locations of Malaysia. BMC Complement Altern Med. 2013, 13, 341), and also 57.25 GAE/g DW of total polyphenol content (Suwannakul, S.; Chaibenjawong, P.; Suwannakul, S. Antioxidant anticancer and antimicrobial activities of ethanol Pandanus amaryllifolius Roxb. leaf extract (In vitro) - A potential medical application. J. Int. Dent. Med. Res. 2018, 11, 383-389).

In the aging process of the skin, free radicals are overproduced, therefore it is beneficial to use substances with antioxidant activity that can eliminate the changes (Tracz, J.; Kaj, K. 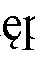 Skóra nie lubi wolnych rodników. Postpy Kosmetologii. 2010, 3, 107-114). It was found that extracts from various parts of *I. glandulifera* (Szewczyk, K.; Bonikowski, R.; Maci 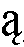 g-Krajewska, A.; Abramek, J.; Bogucka-Kocka, A. Lipophilic components and evaluation of the cytotoxic and antioxidant activities of *Impatiens glandulifera* Royle and *Impatiens noli-tangere* L. (Balsaminaceae). Grasas Y Aceites. 2018, 69, 270; Szewczyk, K.; Kalemba, D.; Komsta, 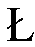 .; Nowak, R. Comparison of the essential oil composition of selected Impatiens species and its antioxidant activities. Molecules. 2016, 21, 1162. https://doi.org/10.3390/molecules21091162; Szewczyk, K.; Sezai Cicek, S.; Zidorn, C.; Granica, S. Phenolic constituents of the aerial parts of Impatiens glandulifera Royle (Balsaminaceae) and their antioxidant activities. Nat. Prod. Res. 2019, 33, 2851-2855; Szewczyk, K.; Zidorn, C.; Biernasiuk, A.; Komsta, 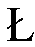 .; Granica, S. Polyphenols from Impatiens (Balsaminaceae) and their antioxidant and antimicrobial activities. Ind. Crops Prod. 2016, 86, 262-272) as well as P. amaryllifolius leaves (Ghasemzadeh, A.; Jaafar, H. Profiling of phenolic compounds and their antioxidant and anticancer activities in pandan (Pandanus amaryllifolius Roxb.) extracts from different locations of Malaysia. BMC Complementary and Alternative Medicine. 2013, 13, 341; Nor, F.; Mohamed, S.; Idris, N.; Ismail, R. Antioxidative properties of Pandanus amaryllifolius leaf extracts in accelerated oxidation and deep frying studies. Food Chem. 2008, 110, 319-327) have strong antioxidant properties.

### Description of Prior Art

*Impatiens balsamina* extract and its use is known from the patent publication CN105267082A. This Impatiens extract exhibits whitening and anti-aging activity and can be used in cosmetics. Furthermore, the invention comprises a method of producing the Impatiens extract and assays including a study of the antioxidant activity of the Impatiens extract (Example 2) and confirming the effects of the Impatiens extract activity (Example 3 -The anti-aging activity assay of the *Impatiens balsamina* extract by, inter alia, determining the elastase activity inhibition index; Example 4 - Tyrosinase inhibition assay).

An antioxidant/anti-allergic agent comprising, inter alia, an extract of *Impatiens textori* petals and/or leaves, in particular a leaf extract, is known from the patent publication JP2013151451A.

A method of obtaining a cosmetic containing an alcoholic solution of *Impatiens balsamina* extract and vitamin C, capable of removing discolorations and freckles, is known from the patent publication JPH10120550A. The cosmetic can whiten the skin thanks to the reducing activity of easily absorbed vitamin C and effectively removes spots and freckles. An alcoholic solution of Impatiens extract is obtained by immersing the whole plant of *Impatiens balsamina* in an aqueous solution containing ethyl alcohol.

DE202013009980U1 discloses essences, cosmetics, pharmaceuticals characterized in that they contain a total of from 9 to 37 original flower essences according to E. Bach, among which the essences derived from, among others, *Impatiens glandulifera* are mentioned.

A skin care preparation is known from the patent JP3591832B2, which is obtained by formulating an extract from the Pandanus L. plant that activates skin fibroblasts and exhibits antioxidant activity, for use in preventing and alleviating the symptoms of skin aging, such

as wrinkles, flaccidity, reduced skin firmness and tarnishing. The formulation is suitable for external application.

JP4445658B2 discloses a composition of a cosmetic with an extract from a plant of the Pandanus genus, the Pandanaceae family (*Pandanus amaryllifolius* Roxb. is a particularly preferred plant). The cosmetic has a skin whitening effect.

A method of extracting volatile components from the leaves of *Pandanus amaryllifolius* Roxb using the supercritical CO₂ extraction method is known from the patent publication CN104083900B. The invention relates to the field of extraction and separation of compounds from the leaves of *Pandanus amaryllifolius* Roxb.

GB201600575 (D0) discloses the use of *Impatients glandulifera* as an active ingredient in antipruritic soaps, antihistamines, shampoos and organic skin lotions.

JP2003342153A discloses a skin care preparation containing one or more plant extracts selected from, inter alia, an aqueous extract of Impatiens (including *I. glandulifera* Royle). JP2003342153A discloses a preparation for activating epidermal cells, activating skin fibroblasts and promoting collagen production. The preparations are intended to maintain the skin in a healthy state and are effective in preventing signs of skin aging, such as wrinkles and deterioration of skin elasticity, caused by aging and UV light.

A method of obtaining an alcohol solution from Impatiens by extraction and the use of this solution in the production of food, soaps and fertilizers is known from the patent publication JP2001025386A.

A method of obtaining plant extract by soaking in alcohol using gallic acid is known from the patent publication JPH0597693A (EP511033A1).

Nowadays, both in the field of scientific research and on the consumer market, there is great interest in raw materials obtained from plants. Plant extracts are obtained from various parts of plants, such as roots, stems, leaves, flowers, seeds or fruits. It is known that they contain valuable compounds for the skin, such as flavonoids, saponins, tannins, mucilages, essential oils, vitamins and nutrients. Extracts can be in liquid or powdered (dry) form. Plant extracts can be a final product or a component of a product used in medicine, cosmetics, etc. They show a wide range of activities, from antibacterial, moisturizing, soothing, alleviating to antioxidant, smoothing or brightening activity.

### Technical Problem

The growing interest of consumers in natural cosmetic products entails the need to look for new methods of obtaining plant extracts, as well as new extracts for use in cosmetics. Extracts from plant material are obtained, for example, by mechanical pressing and by the traditional solvent extraction process. The disadvantages of mechanical pressing are the low efficiency of the process, undesirable enzymatic reactions, and also the risk of oil oxidation. Solvent extraction, on the other hand, poses a risk of contamination of the product with harmful organic compounds, and the use of high temperatures may lead to the loss of bioactive substances, and it is not an environmentally friendly process.

Therefore, it is necessary to search for new solutions allowing to obtain plant extracts in environmentally friendly processes that reduce process time and are economically beneficial. It should be mentioned that the type of extraction is determined depending on the plant species and the active compounds to be obtained for cosmetic and health purposes.

### Solution to Problem

In the developed solution, the plant extract is obtained using an alternative to the above-mentioned methods, namely ultrasound-assisted solvent extraction. It relies on placing the input material (starting material) together with the extractant in an ultrasonic bath. The method generally relies on the fact that the ultrasonic waves cause wave-like pressure changes, which significantly accelerate the extraction process. The phenomenon facilitating the extraction in this case is cavitation, i.e. the formation and disappearance of vapor-gas bubbles. When these bubbles burst, they generate high pressure and temperature, which promotes the penetration of the solvent. The advantages of ultrasound-assisted extraction in obtaining plant extracts are the short duration of the process, the possibility of conducting the process at room temperature, high solubility and diffusion of the solvent.

The use of the ultrasound technique gave more satisfactory effects in comparison to other extraction methods. As a result of breaking the continuity of cells and reducing their size under the influence of ultrasound, the contact surface of the solvent with the sample increases and the desired active compounds are washed out from the cells much easier in comparison to other methods.

When examining the efficiency of ultrasound-assisted extraction, it was unexpectedly found that using the optimal extractant, as well as optimal parameters of the ultrasonic bath and extraction time, an extract with a higher content of polyphenols responsible for the biological activity of the raw material is obtained.

Therefore, the aim of the present invention is to provide a dry and liquid extract of *Impatiens glandulifera* with the addition of *Pandanus amaryllifolius* with a high polyphenol content, a method of its obtaining and use thereof in cosmetics and dietary supplements.

In particular, the aim of the invention is to provide this extract in a liquid form which is a glycerol extract from the flowers, leaves and/or seed pods of *I. glandulifera* with the addition of *P. amaryllifolius* leaves. This extract will show many beneficial properties, important for the anti-aging activity on the skin, antioxidant, anti-inflammatory, skin brightening (whitening) and anti-wrinkle activities, which has been confirmed by research.

### Disclosure of the Invention

According to the present invention, an extract of flowers, leaves and/or seed pods of Impatiens and leaves of Pandanus, in dry and/or liquid form, is characterized in that it is obtained from *Impatiens glandulifera* with the addition of *Pandanus amaryllifolius,* wherein the extract is characterised by a total polyphenol content ranging from 13.0 to 51.0 mg of gallic acid equivalent per g of dry extract.

Preferably, the extract according to the invention contains:

| **No.** | **Compound** | **Content [mg/g of dry extract]** |
|---|---|---|
| 1 | *p*-hydroxybenzoic acid | 18.32 - 20.05 |
| 2 | chlorogenic acid | 3.27 - 3.72 |
| 3 | azelaic acid | 20.89 - 21.33 |
| 4 | *p*-coumaric acid | 2.19 - 2.61 |
| 5 | ferulic acid | 3.52 - 3.93 |
| 6 | vanillic acid | 4.50 - 4.71 |
| 7 | salicylic acid | 2.54 - 2.83 |
| 8 | kaempferol 3-*O*-rutinoside | 2.38 - 2.61 |
| 9 | hyperoside | 1.73 - 1.90 |
| 10 | isoquercitrin | 2.65 - 2.88 |
| 11 | quercitrin | 2.26 - 2.59 |
| 12 | kaempferol 7-*O*-glucoside | 3.93 - 4.26 |
| 13 | astragalin | 7.24 - 7.81 |
| 14 | kaempferol | 11.99 - 13.08 |

Preferably, the extract according to the invention is in the form of a dry extract as a lyophilisate.

Preferably, the extract according to the invention is in a liquid form which is a glycerol solution, preferably in the proportion of 1 part by weight of dry extract and 100-250 parts by weight of glycerol.

Furthermore, the invention comprises a method of obtaining an extract of *Impatiens glandulifera* with the addition of *Pandanus amaryllifolius,* as defined above, characterized in that it comprises the following steps:
(a). providing a material in the form of 9.5-12.0 parts by weight of *Impatiens glandulifera* flowers, leaves and/or seed pods and 0.5-1.0 part by weight of *Pandanus amaryllifolius* leaves;
(b). subjecting the material from (a) to ultrasonic extraction in a bath at the power of 360 W, the frequency of 45 kHz, in 55-100 parts by weight of a 60-90% aqueous ethanol solution and at the temperature of 20-70°C, for 15-90 minutes, producing the aqueous-ethanolic fraction of the extract;
(c). subjecting the aqueous-ethanolic fraction of the extract obtained in (b) to filtration,
(d). concentration of the product obtained in (c), lyophilization and storage below room temperature thereof, with obtaining a dry extract form as a lyophilisate, optionally
(e). dissolving the dry extract obtained in (d) in vegetable glycerol in the proportion of 1 part by weight of dry extract and 500-1250 parts by weight of vegetable glicerol, resulting in the liquid form of this extract.

Preferably, in the method according to the invention, in (a) a material comprising 3 parts by weight of flowers, 6-8 parts by weight of leaves, 0.5-1.0 part by weight of seed pods of *Impatiens glandulifera* and 0.5-1.0 part by weight of leaves of *Pandanus amaryllifolius* is used.

Preferably, in the method according to the invention, the extraction in (b) is carried out in 60-90 parts by weight of a 70-80% aqueous ethanol solution.

Preferably, in the method according to the invention, the extraction in (b) is carried out at 40-50°C.

Preferably, in the method according to the invention, the extraction in (b) is carried out for 30-40 minutes.

Preferably, in the method according to the invention, the lyophilized extract from (d) is stored at a temperature below 10°C.

Preferably, in the method according to the invention, in (e) 900-1150 parts by weight of vegetable glycerol are used per 1 part by weight of dry extract.

The invention also includes a plant extract of *Impatiens glandulifera* with the addition of *Pandanus amaryllifolius,* as defined above, which is obtained by the method as above.

The invention also includes an use of the extract of *Impatiens glandulifera* and *Pandanus amaryllifolius,* as defined above, as an agent having antioxidant, skin brightening and anti-wrinkle properties, inhibiting the activity of collagenase, elastase and tyrosinase, either in cosmetics or in dietary supplements.

Preferably, according to the use, the invention is present in anti-wrinkle creams, preferably in the amount of 4% by weight per 100% by weight of the total composition.

### Brief Description of the Drawing

The object of the invention is shown in the embodiments, with reference to the attached Figures, in which:
**Fig. 1** shows the viability of human skin fibroblasts after 24 hours incubation with the extract of Example 1. The MTT assay was performed to assess viability. *Statistically significant difference compared to control cells - incubated without the studied extract (0 µg/mL); *p <* 0.05, t-test';
**Fig. 2** shows the chromatogram of the quantitative analysis of the identified compounds 1-14 (shown in Table 6) prepared on the basis of calibration curves obtained for the respective standards, where the x-axis is time in minutes and the y-axis is relative abundance, and
**Fig. 3** shows a visual representation of the results of the wrinkle width measurements, showing the location of the tested area before a start of using (D0) and after 28 days (D28) of regular product application, measured in mm.

### Detailed Description of the Invention

The subject matter of the present invention is described in detail below with reference to the attached Figures and Examples.

Generally, the method of obtaining an extract of *I. glandulifera* and *P. amaryllifolius* in a dry form according to the invention relies on using *I. glandulifera* flowers, leaves and seed pods and *P. amaryllifolius* leaves as the starting material and their extraction in an ultrasonic bath (Walter Powersonic P 1500 D , Martin Walter Ultraschalltechnik) in an aqueous ethanol solution (Chempur Poland) in the proportion of 3 parts by weight of flowers, 6 to 8 parts by weight of leaves, 0.5 to 1 part by weight of Impatiens seed pods, 0.5 to 1.0 part by weight of Pandanus leaves and 55-100 parts by weight of a 60-90% ethanol solution, preferably 60-90 parts by weight of a 70-80% ethanol solution, at a temperature of 20-70°C, preferably 40-50°C, for 15-90 minutes, preferably 30-40 minutes. Then, the water-ethanol fraction is filtered, and the obtained extract is concentrated, lyophilized and stored at a temperature below room temperature, preferably at the temperature below 10°C, with obtaining a dry extract form (lyophilisate) of *I. glandulifera* and *P. amaryllifolius.*

In addition, the subject of the invention is also an extract according to the invention in liquid form, which is a glycerol extract of the flowers, leaves and seed pods of *I. glandulifera* and *P. amaryllifolius* leaves, obtained by dissolving the dry extract, obtained as above, in vegetable glycerol in the proportion of 1 part by weight of dry extract and 500-1250 parts by weight of vegetable glycerol (Chemitec Poland), preferably 900-1150 parts by weight of glycerol.

### Examples

Hereinafter, the present invention will be illustrated via working examples.

### Example 1

30 g of flowers, 60 g of leaves and 10 g of seed pods of *I. glandulifera* and 5 g of leaves of *P. amaryllifolius* were placed in the Erlenmayer flask and 600 mL of 70% ethanol (Chempur Poland) was added, and then extracted in an ultrasonic bath (Walter Powersonic P 1500 D, Martin Walter Ultraschalltechnik), at the power of 360 W, the frequency of 45 kHz, at the temperature of 45°C for 30 minutes. Then, the solid fraction was separated by filtration using a paper filter, the aqueous-alcoholic fraction was concentrated under reduced pressure, and then lyophilised. 16.233 g of lyophilisate (process yield 15.46%) of *I. glandulifera* and *P*. *amaryllifolius* extract was obtained, in which 48.69 ± 2.19 mg GAE/g of dry extract of total polyphenol content, expressed as gallic acid equivalent, was found. The lyophilised extract was characterized by 81.73% activity inhibition of collagenase from *Clostridium haemolyticum* (18.27% of initial collagenase activity), 83.19% inhibition of elastase (16.81% of initial elastase activity), 73.38% inhibition of soybean lipoxygenase, and 53.95% inhibition of fungal tyrosinase at the extract concentration of 100 µg/mL of glycerol. In addition, the lyophilised extract at a concentration of 1.95-250 µg/mL of glycerol showed no cytotoxic effect against human skin fibroblasts.

### Example 2

10 g of flowers, 80 g of leaves and 5 g of seed pods of *I. glandulifera* and 10 g of leaves of *P. amaryllifolius* were placed in the Erlenmayer flask and 600 mL of 70% ethanol was added, and then extracted in an ultrasonic bath at the power of 360 W, the frequency of 45 kHz, at the temperature of 45°C for 30 minutes. Then, the solid fraction was separated by filtration using a paper filter, the aqueous-alcoholic fraction was concentrated under reduced pressure, and then lyophilised. 12.725 g of lyophilisate was obtained (process yield 12.12%), in which 26.56 ± 1.27 mg GAE/g of dry extract of total polyphenol content, expressed as gallic acid equivalent, was found. The lyophilised extract was characterized by 63.05% activity inhibition of collagenase from *Clostridium haemolyticum,* 42.59% inhibition of elastase, 48.10% inhibition of soybean lipoxygenase, and 25.72% inhibition of fungal tyrosinase at the extract concentration of 100 µg/mL of glycerol.

### Example 3

30 g of flowers, 60 g of leaves and 10 g of seed pods of *I. glandulifera* and 5 g of leaves of *P. amaryllifolius* were placed in the Erlenmayer flask and 600 mL of 50% ethanol was added, and then extracted in an ultrasonic bath at the power of 360 W, the frequency of 45 kHz, at the temperature of 45°C for 30 minutes. Then, the solid fraction was separated by filtration using a paper filter, the aqueous-alcoholic fraction was concentrated under reduced pressure, and then lyophilised. 9.167 g of lyophilisate was obtained (process yield 8.73%), in which 13.10 ± 0.05 mg GAE/g of dry extract of total polyphenol content, expressed as gallic acid equivalent, was found. The lyophilized extract was characterized by 21.09% activity inhibition of collagenase from *Clostridium haemolyticum,* 7.58% inhibition of elastase, and 16.26% inhibition of soybean lipoxygenase at the extract concentration of 100 µg/mL of glycerol.

### Example 4

30 g of flowers, 60 g of leaves and 10 g of seed pods of *I. glandulifera* and 5 g of leaves of *P. amaryllifolius* were placed in the Erlenmayer flask and 600 mL of 70% ethanol was added, and then extracted in an ultrasonic bath at the power of 240 W, the frequency of 45 kHz, at the temperature of 45°C for 30 minutes. Then, the solid fraction was separated by filtration using a paper filter, the aqueous-alcoholic fraction was concentrated under reduced pressure, and then lyophilised. 13.422 g of lyophilisate was obtained (process yield 12.78%), in which 33.36 ± 0.42 mg GAE/g of dry extract of total polyphenol content, expressed as gallic acid equivalent was found. The lyophilized extract was characterized by 67.18% activity inhibition of collagenase from *Clostridium haemolyticum,* 35.09% inhibition of elastase, 49.59% inhibition of soybean lipoxygenase, and 19.72% inhibition of fungal tyrosinase at the extract concentration of 100 µg/mL of glycerol.

### Example 5

30 g of flowers, 60 g of leaves and 10 g of seed pods of *I. glandulifera* and 5 g of leaves of *P. amaryllifolius* were placed in the Erlenmayer flask and 600 mL of 70% ethanol was added, and then extracted in an ultrasonic bath at the power of 360 W, the frequency of 45 kHz, at the temperature of 20°C for 30 minutes. Then, the solid fraction was separated by filtration using a paper filter, the aqueous-alcoholic fraction was concentrated under reduced pressure, and then lyophilised. 8.617 g of lyophilisate was obtained (process yield 8.21%), in which 18.07 ± 0.09 mg GAE/g of dry extract of total polyphenol content, expressed as gallic acid equivalent, was found. The lyophilized extract was characterized by 70.28% activity inhibition of collagenase from *Clostridium haemolyticum,* 54.19% inhibition of elastase, 58.25% inhibition of soybean lipoxygenase, and 38.50% inhibition of fungal tyrosinase at the extract concentration of 100 µg/mL of glycerol.

### Example 6

30 g of flowers, 60 g of leaves and 10 g of seed pods of *I. glandulifera* were placed in the Erlenmayer flask and 600 mL of 70% ethanol was added, and then extracted in an ultrasonic bath at the power of 360 W, the frequency of 45 kHz, at the temperature of 45°C for 30 minutes. Then, the solid fraction was separated by filtration using a paper filter, the aqueous-alcoholic fraction was concentrated under reduced pressure, and then lyophilised. 14.735 g of lyophilisate (process yield 14.74%) of *I. glandulifera* extract was obtained, in which 45.72 ± 0.30 mg GAE/g of dry extract of total polyphenol content, expressed as gallic acid equivalent, was found.

### Example 7

The subject of the invention is also the use of an extract from the flowers, leaves and seed pods of *I. glandulifera* and the leaves of *P. amaryllifolius* for obtaining cosmetic preparations, particularly anti-wrinkle preparations, obtained as a result of extraction in an ultrasonic bath as in Example 1. Examplary formulations of the preparations:

### 1. Day cream (anti-wrinkle)

| Phase A | | |
|---|---|---|
| GSC | Glyceryl Stearate Citrate | 5.0% |
| Cetyl alcohol | | 2.0% |
| Behenyl alcohol | | 0.5% |
| Jasmine butter | | 1.5% |
| Cetiol ultimate | Undecane (and) Tridecane | 1.5% |
| Caprylic-capric triglyceride | | 2.0% |
| Isopropyl myristate | | 1.0% |
| Cetiol sensoft | Octanoic acid, 2-propylheptyl ester | 1.0% |
| Ethylhexyl Octanoate | 2-ethylhexyl acetate | 2.0% |

| Phase B | | |
|---|---|---|
| Propanediol | | 1.5% |
| Polymer thickener | | 1.0% |
| Water | | 68.0% |

| Phase C | | |
|---|---|---|
| Extract of Example 1 (100 µg/mL of glycerol) | | 4.0% |
| Raspberry oil | | 2.0% |
| Prickly pear oil | | 2.0% |
| Apple stem cells | | 2.0% |
| Vitamin E | | 1.0% |
| Preservative | | 1.0% |
| Panthenol | | 1.0% |
| Fragrance | | q.s. |

### Preparation:

Heat the ingredients of phase A in a water bath until they dissolve, mix the ingredients of phase B and heat to the temperature of phase A. Add the warm phase A to the warm phase B and mix until a creamy consistency is obtained. Add the phase C ingredients to the cooled preparation and then homogenise using a homogeniser.

### 2. Night cream (anti-wrinkle)

| Phase A | | |
|---|---|---|
| Decaglyn 10-SV | Polyglyceryl-10 decastearate | 4.0% |
| Glyceryl monostearate | | 1.5% |
| Cetyl alcohol | | 2.0% |
| Behenyl alcohol | | 1.0% |
| Jasmine butter | | 2.0% |
| Macadamia butter | | 1.0% |
| Cetiol ultimate | Undecane (and) Tridecane | 2.0% |
| Caprylic-capric triglyceride | | 2.0% |
| Isopropyl myristate | | 1.5% |
| Cetiol sensoft | Octanoic acid, 2 propylheptyl ester | 2.0% |
| Ethylhexyl octanoate | 2-ethylhexyl acetate | 2.0% |

| Phase B | | |
|---|---|---|
| Propanediol | | 2.0% |
| Polymer thickener | | 1.0% |
| Water | | 65.0% |

| Phase C | | |
|---|---|---|
| Extract of Example 1 (100 µg/mL of glycerol) | | 4.0% |
| Macadamia oil | | 2.0% |
| Argan oil | | 1.0% |
| Apple stem cells | | 3.0% |
| Preservative | | 1.0% |
| Fragrance | | q.s. |

### Preparation:

Heat the ingredients of phase A in a water bath until they dissolve, mix the ingredients of phase B and heat to the temperature of phase A. Add the warm phase A to the warm phase B and mix until a creamy consistency is obtained. Add the ingredients of phase C to the cooled preparation and then homogenise using a homogeniser.

### Assays confirming the acivity of the extract according to the invention

According to the invention, the extract of Example 1 exhibits a number of beneficial properties relevant to anti-aging effect. The following properties have been proven for it:

### 1. Inhibition of collagenase activity isolated from Clostridium haemolyticum

The enzymatic method for the determination of collagenase (EC 3.4.24.3) using FALGPA (N-(3-[2-furyl]acryloyl)-Leu-Gly-Pro-Ala) as substrate for collagenase, which was dissolved in 50 mM Tricine buffer (pH 7.5) at an initial concentration of 0.8 U/mL, was used for the study. FALGPA was dissolved in Tricine buffer to 2 mM. Various concentrations were prepared from the lyophilised extract of Example 1 by dissolving in glycerol and incubating with the enzyme for 15 minutes before adding the substrate to start the reaction. The final reaction mixture (150 µL) contained Tricine buffer, 0.8 mM of FALGPA, 0.1 U of collagenase and the extract at appropriate concentration. Epigallocatechin gallate (EGCG; Supelco) at the concentration of 100 µg/mL was used as a standard. The absorbance at wavelength of λ=345 nm was measured immediately after substrate addition and then continuously for 20 minutes. The results are presented in Table 1 below.

**Table 1**

| **Concentration of lyophilisate of Example 1 in µg/mL of glycerol** | **Collagenase inhibition (%)** |
|---|---|
| 5 | 9.28 |
| 10 | 28.07 |
| 25 | 43.37 |
| 50 | 13.81 |
| 100 | 81.73 |
| EGCG | 84.45 |

The strongest inhibition showed the extract of Example 1 at a concentration of 100 µg/mL of glycerol and it was similar to the activity of EGCG used as a standard. The results demonstrate a very high inhibitory capacity of collagenase from *Clostridium haemolyticum* activityby the extract according to the invention.

### 2. Inhibition of elastase activity

The method using porcine pancreatic elastase (PE - E.C.3.4.21.36; Sigma-Aldrich) was used for the study, which was dissolved in distilled water to prepare a stock solution with a concentration of 3.33 mg/mL. The substrate N-Succinyl-Ala-Ala-p-nitroanilide (AAAPVN; Sigma-Aldrich) was dissolved in 1.6 mM Tris-HCl buffer (pH 8.0). The extract of Example 1 dissolved in glycerol (concentrations of 5-100 µg/mL) was incubated with the enzyme for 15 minutes before adding the substrate to start the reaction. The final reaction mixture in the well of the 96-well plate (total volume 200 µL) contained buffer, 0.8 mM of AAAPVN, 1 µg/mL of elastase and 25 µL of extract at the appropriate concentration. Epigallocatechin gallate (EGCG; Supelco) at the concentration of 100 µg/mL was used as a standard. Negative controls were performed using water. Absorbance values were measured immediately after substrate addition and then after 20 minutes at wavelength of λ=410 nm. The results are presented in Table 2 below.

**Table 2**

| **Concentration of lyophilisate of Example 1 in µg/mL of glycerol** | **Elastase inhibition (%)** |
|---|---|
| 5 | 0 |
| 10 | 25.62 |
| 25 | 40.77 |
| 50 | 57.31 |
| 100 | 83.19 |
| EGCG | 92.25 |

The strongest inhibition showed the extract of Example 1 at a concentration of 100 µg/mL and it was the activity similar to the EGCG acivity used as a standard. The results demonstrate a very high inhibitory capacity of elastase activity by the extract according to the invention.

### 3. Antioxidant properties

An assessment of the antioxidant properties was conducted by determining the ability of the lyophilised extract of Example 1 to neutralise the violet radical 2,2-diphenyl-1-picrylhydrazyl (DPPH•, Sigma-Aldrich). An ethanolic solution of DPPH• at the concentration of 0.07 mg/mL was prepared for the assays, as well as a glycerol stock solution of the extract at the concentration of 1 mg/mL. Twenty µL of the extract at various concentrations and 180 µL (each) of the DPPH• solution at a concentration of 0.07 mg/mL were applied to 96-well plates (Googlab Professional Line). Then incubation for 30 minutes in the dark at room temperature was conducted, after which the absorbance was measured at wavelength of λ=517 nm. While in the control sample, instead of the extract, 20 µL of glycerol was applied to the well. On the basis of the obtained results, the antioxidant activity was calculated, which was expressed as Trolox equivalent (Sigma-Aldrich) in µg/mL. The results are presented in Table 3 below.

**Table 3**

| **Concentration of lyophilisate of Example 1 according to the invention in µg/mL of glycerol** | **Antioxidant activity [µg Trolox/mL]** |
|---|---|
| 5 | 55.22 |
| 10 | 99.55 |
| 25 | 133.58 |
| 50 | 173.91 |
| 100 | 177.07 |

The results were compared with the activity of Trolox, with the known high antioxidant activity, and are presented as its equivalent. The results demonstrate a very good antioxidant activity of the extract according to the invention.

### 4. Anti-inflammatory properties

The study of anti-inflammatory activity of the extract of Example 1 was assessed on the basis of lipoxygenase inhibition using a spectrophotometric method in 96-well plates. For this purpose, 100 µL of various concentrations of extract solution in potassium phosphate buffer (pH = 8.5), 50 µL of soybean lipoxygenase solution (LOX; 167 U/mL; Sigma-Aldrich Chemie GmbH, Steinheim, Germany) in potassium phosphate buffer (pH = 8.5), and finally 50 µL of linoleic acid solution (LA; 134 µM; Sigma-Aldrich Chemie GmbH, Steinheim, Germany) were added to the wells. Changes in absorbance at 234 nm were then measured for a period of 25 minutes with intervals of 30 seconds. Indomethacin (Sigma-Aldrich) was used as a positive control. The activity of the extract was calculated as the percentage of LOX enzyme activity (% of activity) in relation to the maximal activity (negative control - solution without studied extract) after subtracting the absorbance of the extract at 234 nm in all cases. Absorbance values at the 10th minute of the reaction were used for the calculations. The results are presented in Table 4 below.

**Table 4**

| **Concentration of the lyophilisate of Example 1 according to the invention in µg/mL of glycerol** | **Inhibition of LOX activity [%]** |
|---|---|
| 5 | 14.02 |
| 10 | 15.59 |
| 25 | 23.96 |
| 50 | 39.15 |
| 100 | 73.38 |
| Indomethacin | 99.80 |

The strongest inhibition showed the extract of Example 1 at a concentration of 100 µg/mL and it was the activity similar to the Indomethacin activity used as a standard. The results demonstrate that the extract according to the invention can be considered as a potent LOX inhibitor.

### 5. Inhibition of fungal tyrosinase activity

The tyrosinase (EC 1.14.18.1, 25 KU, fungal tyrosinase, Sigma-Aldrich) inhibitory effect was measured using L-3,4-dihydroxyphenylalanine (L-DOPA, Supelco) as a substrate. To perform it, various concentrations of the studied extract (20 µL), tyrosinase solution (200 U/mL, 40 µL) and phosphate buffer (100 µL, 50 mM, pH 6.5) were applied to a 96-well plate, and then incubated for 15 minutes at 25°C. The reaction was started with the addition of L-DOPA (40 µL) and incubated for a further 15 minutes, after which the absorbance was measured at wavelength of λ=475 nm compared to the control without the inhibitor. In the control sample, representing 100% of enzyme activity, phosphate buffer was added instead of the extract. Tyrosinase inhibitory activity was determined by comparing the enzyme activity in samples with and without the assessed inhibitor. Kojic acid (Supelco) at a concentration of 100 µL/mL was used as a standard. The results are presented in Table 5 below.

**Table 5**

| **Concentration of lyophilisate of Example 1 in µg/mL of glycerol** | **Tyrosinase inhibition [%]** |
|---|---|
| 5 | 12.17 |
| 10 | 30.38 |
| 25 | 36.91 |
| 50 | 42.18 |
| 100 | 53.95 |
| kojic acid | 61.17 |

Tyrosinase is the rate-limiting enzyme for melanin production. The anti-tyrosinase effect was determined by calculating the oxidation of L-DOPA to dopaquinone in an in vitro fungal tyrosinase assay. The strongest inhibition showed the extract at a concentration of 100 µg/mL and it was the activity similar to the reference substance activity, which was kojic acid. The strong anti-tyrosinase activity of the extract can be attributed to the high total polyphenol content (48.69 mg GAE/g of extract), which not only have a good affinity for tyrosinase preventing the formation of dopaquinone, but can also bind to it and reduce its catalytic activity (Chang, T.S. (2009). An updated review of tyrosinase inhibitors. International Journal of Molecular Sciences, 10, 2440-2475. doi:10.3390/ijms10062440). It is known that some phenolic compounds, such as kaempferol and quercetin described in the Impatiens genus, are potent tyrosinase inhibitors (Kim, Y. J., Uyama, H. (2005). Tyrosinase inhibitors from natural and synthetic source: Structure, inhibition mechanism and prospective for the future. Cellular and Molecular Life Sciences, 62, 1707-1723. doi:10.1007/s00018-005-5054-y; Solano, F., Briganti, S., Picardo, M., Ghanem, G. (2006). Hypopigmenting agents: an updated review on biological, chemical and clinical aspects. Pigment Cell Research, 19, 550-571. doi:10.1111/j.1600-0749.2006.0 0334.x; Hua, L., Peng, Z., Chia, L. S., Goh, N. K., Tan, S. N. (2001). Separation of kaempferols in Impatiens balsamina flowers by capillary electrophoresis with electrochemical detection. Journal of Chromatography A, 909, 297-303. doi:10.1016/S0021-9673(00)01102-X; Szewczyk, K., Sezai Cicek, S., Zidorn, C., Granica, S. (2019). Phenolic constituents of the aerial parts of Impatiens glandulifera Royle (Balsaminaceae) and their antioxidant activities. Natural Product Research, 33, 2851-2855; Jiang, H.F., Zhuang, Z.H., Hou, B.W., Shi, B.J., Shu, C.J., Chen, L., Shi, G.X., Zhang, W.M. 2017. Adverse effects of hydroalcoholic extracts and the major components in the stems of Impatiens balsamina L. on Caenorhabditis elegans. Evid. Based. Complement. Alternative Med. 2017. https://doi.org/10.1155/2017/4245830; Lim, Y.H., Kim, I.H., Seo, J.J., 2007. In vitro activity of kaempferol isolated from the Impatiens balsamina alone and in combination with erythromycin or clindamycin against Propionibacterium acnes. J. Microbiol. 45, 473-477).

### 6. Cytotoxicity assessment

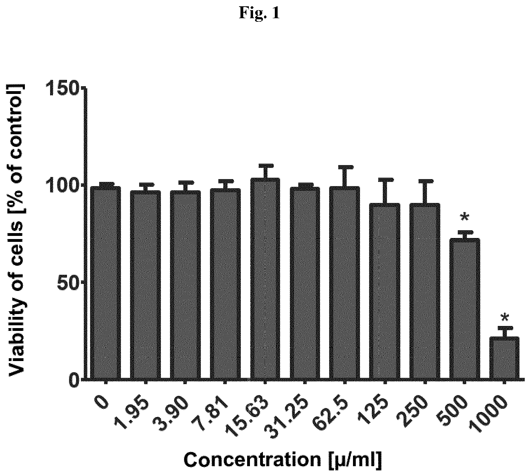

The cytotoxicity assessment of the extract of Example 1 was performed using a human normal skin fibroblast (BJ) cell line (American Cell Culture Collection - ATCC). The BJ cells were seeded in 96-well plates at a concentration of 2x10⁴ cells/well and incubated for 24 hours at the temperature of 37°C. The next day the extract of Example 1 was dissolved in DMSO. The concentration of the stock solution was 100 mg/mL. Dilutions of the studied extract in culture medium (1000-1.95 µg/mL DMSO) were then prepared and added to the cells. The plates were incubated for 24 hours at the temperature of 37°C. The fibroblasts viability after incubation with the studied extract was assessed using the MTT assay (Sigma-Aldrich). Control cells were incubated in culture medium without the studied extract (0 µg/mL DMSO). The results are presented in Fig. 1 below, where Fig. 1 shows the viability of human skin fibroblasts after 24 hours of incubation with the extract of Example 1. The MTT assay was performed to assess the viability. *Statistically significant difference compared to the control cells - incubated without the studied extract (0 µg/mL DMSO); p < 0.05, t-test.

The conducted experiment showed (Fig. 1) that the extract of Example 1 at concentrations of 1.95-250 µg/mL showed no cytotoxic effect against human skin fibroblasts. A statistically significant decrease of viability compared to control cells (p < 0.05) was noticed after incubation with the extract at concentrations of 500 µg/mL and 1000 µg/mL. Cell viability was reduced to approximately 72% (500 µg/mL concentration) and to approximately 21% (1000 µg/mL concentration), respectively.

### 7. Analysis of the content of the main active compounds

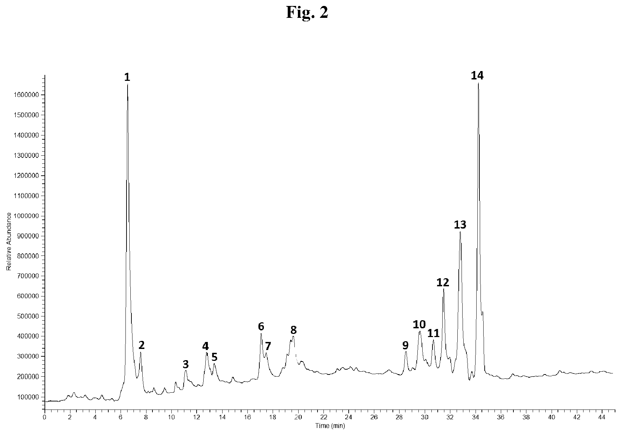

The qualitative and quantitative analysis of the main active compounds contained in the extract of Example 1 was performed using liquid chromatography coupled with the LC/MS mass spectrometry (Thermo Scientific; Q-EXATCTIVE and ULTIMATE 3000, San Jose, CA) equipped with an ESI electrospray source. The ESI was operated in a positive polarity mode under the following conditions: electric field voltage: 3.5 kV; flow rate of N₂ dispersing gas (>99.98%): 40 arb.; N₂ drying gas (>99.98%): 10 arb.; capillary temperature: 320 °C. A Gemini C18 column (4.6 × 100 mm, 3 µm) (Phenomenex, USA) was used for chromatographic separation, which was conducted using gradient elution. Mobile phase A contained 25 mM of formic acid in water; mobile phase B was 25 mM formic acid in acetonitrile. The gradient program started at 5% B increasing to 95% for 60 minutes, followed by isocratic elution (95% B) for 10 minutes. The total run time was 70 minutes at the mobile phase flow rate of 0.4 mL/min. The column temperature was 25°C. In the course of each run, MS spectra in the range of 100 - 700 m/z were collected continuously. A quantitative analysis of the identified compounds was made on the basis of calibration curves obtained for the respective standards. The results are presented in the chromatogram below (Fig. 2) and in Table 6.

**Table 6**

| **No.** | **Compound** | **Content [mg/g of dry extract according to the invention]** |
|---|---|---|
| 1 | *p*-hydroxybenzoic acid | 19.19 ± 0.61 |
| 2 | chlorogenic acid | 3.57 ± 0.14 |
| 3 | azelaic acid | 21.04 ± 0.69 |
| 4 | *p*-coumaric acid | 2.45 ± 0.09 |
| 5 | ferulic acid | 3.78 ± 0.15 |
| 6 | vanillic acid | 4.57 ± 0.17 |
| 7 | salicylic acid | 2.67 ± 0.09 |
| 8 | kaempferol 3-*O*-rutinoside | 2.49 ± 0.08 |
| 9 | hyperoside | 1.81 ± 0.06 |
| 10 | isoquercitrin | 2.78 ± 0.10 |
| 11 | quercitrin | 2.47 ± 0.09 |
| 12 | kaempferol 7-*O*-glucoside | 4.14 ± 0.16 |
| 13 | astragalin | 7.57 ± 0.29 |
| 14 | kaempferol | 12.41 ± 0.55 |

### 8. Dermatological study with the semi-open ordinary test

The aim of the study was to evaluate the irritant properties (dermal tolerance) of the extract on healthy skin of adult, using a patch test. The volunteers (25 people) were healthy, with a negative history of allergy. Group selection included inclusion and exclusion criteria. Main inclusion criteria: healthy men and women from the age of 18 years and older, phototype: I-IV on the Fitzpatrick scale, Caucasian skin type, skin without irritation and lesions requiring treatment. Main exclusion criteria: existing skin lesions requiring treatment in the study area, volunteers with a history of dermatological diseases or serious chronic diseases that may affect the study results, pregnant or breastfeeding women and those planning to become pregnant during the study. None of the volunteers reported a documented hypersensitivity or a history of adverse reactions to individual components of the study product. All volunteers fulfilled the inclusion requirements for the trials and signed an informed consent form (ICF). In addition, they were informed of the purpose, study methodology and possible adverse effects. The skin at the application site (arms or interscapular area) was healthy, without lesions. The volunteers were informed of the necessity and special precautions in handling the applied contact tests.

The extract in the usable concentration was applied to 12 mm diameter filter paper discs from SmartPractice^{®} and then adhered to the arm or interscapular area with a patch. In order to objectify the results and exclude possible reading errors related to skin irritation, two control samples were used (a 'blank' sample and a sample using water). Then, 48 hours after application, the dermatologist peeled off the patch and took a reading 30 minutes after removing the patch. 72 hours after application, the dermatologist checked the skin reaction again. If irritation appears or persists 72 hours after application, an additional reading is taken after 96 hours. By reading the skin reaction, the dermatologist assesses the irritating and sensitizing activity of the studied product. The study results can be influenced by factors such as lifestyle, stress, diet and environmental conditions, etc. The study results are presented in Table 7 below.

**Table 7. Study results of volunteers with a negative history of allergy.**

| **Volunteer No.** | **Study results after 48 hours of sample application** | | **Study results after 73 hours of sample application** | | **Study results after 96 hours of sample application** | |
|---|---|---|---|---|---|---|
| | **erythema** | **oedema** | **erythema** | **oedema** | **erythema** | **oedema** |
| 1 | 0 | 0 | 0 | 0 | Study omitted | |
| 2 | 0 | 0 | 0 | 0 | Study omitted | |
| 3 | 0 | 0 | 0 | 0 | Study omitted | |
| 4 | 0 | 0 | 0 | 0 | Study omitted | |
| 5 | 0 | 0 | 0 | 0 | Study omitted | |
| 6 | 0 | 0 | 0 | 0 | Study omitted | |
| 7 | 0 | 0 | 0 | 0 | Study omitted | |
| 8 | 0 | 0 | 0 | 0 | Study omitted | |
| 9 | 0 | 0 | 0 | 0 | Study omitted | |
| 10 | 0 | 0 | 0 | 0 | Study omitted | |
| 11 | 0 | 0 | 0 | 0 | Study omitted | |
| 12 | 0 | 0 | 0 | 0 | Study omitted | |
| 13 | 0 | 0 | 0 | 0 | Study omitted | |
| 14 | 0 | 0 | 0 | 0 | Study omitted | |
| 15 | 0 | 0 | 0 | 0 | Study omitted | |
| 16 | 0 | 0 | 0 | 0 | Study omitted | |
| 17 | 0 | 0 | 0 | 0 | Study omitted | |
| 18 | 0 | 0 | 0 | 0 | Study omitted | |
| 19 | 0 | 0 | 0 | 0 | Study omitted | |
| 20 | 0 | 0 | 0 | 0 | Study omitted | |
| 21 | 0 | 0 | 0 | 0 | Study omitted | |
| 22 | 0 | 0 | 0 | 0 | Study omitted | |
| 23 | 0 | 0 | 0 | 0 | Study omitted | |
| 24 | 0 | 0 | 0 | 0 | Study omitted | |
| 25 | 0 | 0 | 0 | 0 | Study omitted | |

After conducting a patch test under dermatological control on a group of 25 volunteers, it was found that the studied extract of Example 1 (100 mg/L of glycerol) used by people who had no documented allergy to any of the ingredients was well tolerated by the skin. No irritation or allergic reactions occurred in the studied group of volunteers. The extract according to the invention therefore meets the requirements of the Skin Compatibility Test and can be classified as non-irritating.

### 9. Instrumental studies of skin moisturization level using the Corneometer^{®} CM 825

The aim of the study was to determine the effect of the extract on skin moisturization level. The study was conducted using a specialised measuring device from Courage & Khazaka Company - Corneometer^{®} CM 825. Eleven volunteers took part in the instrumental study. The study was performed at the application area before (D0) and after 28 days (D28) of regular application of the extract according to the invention. The study was conducted in an air-conditioned room with a temperature of 20±2°C and a relative humidity of 50±10%. The activity of the extract is confirmed in the case of positive results obtained in more than 50% of the study participants. The results are presented in Table 8 below.

**Table 8. Corneometer^{®} CM 825. Results of skin moisturisation measurements performed before a start of using (D0) and after 28 days (D28) of regular product application in corneometer unit.**

| **Volunteer No.** | **Before (D0)** | **After 28 days (D28)** | **Difference (D28-D0)** |
|---|---|---|---|
| 1 | 44.2 | 45.3 | 1.1 |
| 2 | 46.9 | 47.8 | 0.8 |
| 3 | 27.7 | 29.5 | 1.8 |
| 4 | 43.5 | 44.4 | 1.0 |
| 5 | 39.2 | 40.0 | 0.8 |
| 6 | 48.3 | 50.4 | 1.1 |
| 7 | 50.0 | 51.3 | 1.3 |
| 8 | 43.1 | 45.0 | 1.9 |
| 9 | 49.0 | 50.1 | 1.1 |
| 10 | 54.1 | 56.2 | 2.1 |
| 11 | 46.4 | 47.0 | 0.6 |
| Mean | 44.9 | 46.1 | 1.2 |
| Min | 27.7 | 29.5 | 0.6 |
| Max | 54.1 | 56.2 | 2.1 |
| SD | 7.0 | 7.0 | 0.5 |
| Median | 46.4 | 47.0 | 1.1 |
| Δ% | | | 3% |
| % of volunteers with positive effect | | | 100% |

On the basis of conducted application studies, after 28 days of regular application, it was found that the extract according to the invention at a concentration of 100 mg/L, moisturises the skin (on average by 3%).

### 10. Instrumental studies of skin smoothing level using the Visioscan^{®} VC 20plus

The aim of the study was to confirm the skin smoothing activity with Visioscan^{®} VC 20plus. The study was conducted using a specialised measuring device from Courage & Khazaka Company. Eleven volunteers took part in the instrumental study. The study was performed at the application area before (D0) and after 28 days (D28) of regular product application. The study was conducted in an air-conditioned room with a temperature of 20±2°C and a relative humidity of 50±10%. The activity of the extract is confirmed in the case of positive results obtained in more than 50% of the study participants. The extract smoothes the skin when the parameter value is reduced. The results are presented in Table 9 below.

**Table 9. Visioscan^{®} VC 20plus. Results of skin smoothing measurements performed before a start of using (D0) and after 28 days (D28) of regular product application in mm³.**

| **Volunteer No.** | **Before (D0)** | **After 28 days (D28)** | **Difference (D28-D0)** |
|---|---|---|---|
| 1 | 117.7 | 92.0 | -25.6 |
| 2 | 97.2 | 93.1 | -4.1 |
| 3 | 93.0 | 85.5 | -7.4 |
| 4 | 98.9 | 89.3 | -9.6 |
| 5 | 69.4 | 68.3 | -1.1 |
| 6 | 130.2 | 107.7 | -22.5 |
| 7 | 105.5 | 107.0 | 1.4 |
| 8 | 93.2 | 90.8 | -2.4 |
| 9 | 104.5 | 103.1 | -1.4 |
| 10 | 94.7 | 82.3 | -12.4 |
| 11 | 116.6 | 98.9 | -17.6 |
| Mean | 101.9 | 92.6 | -9.3 |
| Min | 69.4 | 68.3 | -25.6 |
| Max | 130.2 | 107.7 | 1.4 |
| SD | 16.1 | 11.6 | 9.2 |
| Median | 98.9 | 92.0 | -7.4 |
| Δ% | | | -9% |
| % of volunteers with positive effect | | | 91% |

On the basis of conducted application studies, after 28 days of regular application, it was found that the extract prepared according to the invention at a concentration of 100 mg/L smoothes the skin (on average by 9%).

### 11. Instrumental studies of skin biomechanical parameters using Cutometer^{®} MPA 580

The aim of the study was to confirm the activity of improving the skin biomechanical parameters using Cutometer^{®} MPA 580. The study was conducted using a specialised measuring device from Courage & Khazaka Company. Eleven participants took part in the efficacy study. The test was performed at the application area before (D0) and after 28 days (D28) of regular product application. The study was conducted in an air-conditioned room with a temperature of 20±2°C and a relative humidity of 50±10%. The activity of the extract is confirmed in the case of positive results obtained in more than 50% of the study participants. The extract improves the skin elasticity (Table 10) when the parameter value increases, and also improves skin firmness (Table 11) when the parameter value decreases.

**Table 10. Cutometer^{®} MPA 580. Measurements of the R2 parameter (skin elasticity) performed before a start of using (D0) and after 28 days (D28) of regular product application.**

| **Volunteer No.** | **Before (D0)** | **After 28 days (D28)** | **Difference (D28-D0)** |
|---|---|---|---|
| 1 | 0.495 | 0.661 | 0.166 |
| 2 | 0.731 | 0.774 | 0.043 |
| 3 | 0.746 | 0.794 | 0.048 |
| (4)* | (0.541)* | AV | |
| 5 | 0.691 | 0.623 | -0.068 |
| 6 | 0.669 | 0.756 | 0.087 |
| 7 | 0.960 | 0.946 | -0.015 |
| 8 | 0.774 | 0.799 | 0.025 |
| 9 | 0.609 | 0.730 | 0.121 |
| 10 | 0.788 | 0.839 | 0.050 |
| 11 | 0.772 | 0.740 | -0.032 |
| Mean | 0.723 | 0.766 | 0.043 |
| Min | 0.495 | 0.623 | -0.068 |
| Max | 0.960 | 0.946 | 0.166 |
| SD | 0.123 | 0.090 | 0.070 |
| Median | 0.738 | 0.765 | 0.045 |
| Δ% | | | 6% |
| % of volunteers with positive effect | | | 70% |

| | | | |
|---|---|---|---|
| Legend: ()* - Result not included in calculation; AV - incorrect value | | | |

**Table 11. Cutometer^{®} MPA 580. Measurements of R0 parameter (skin firmness) performed before a start of using (D0) and after 28 days (D28) of regular product application in mm.**

| **Volunteer No.** | **Before (D0)** | **After 28 days (D28)** | **Difference (D28-D0)** |
|---|---|---|---|
| 1 | 0.485 | 0.524 | 0.039 |
| 2 | 0.475 | 0.366 | -0.110 |
| 3 | 0.201 | 0.167 | -0.034 |
| (4)* | (0.412)* | AV | |
| 5 | 0.223 | 0.230 | 0.007 |
| 6 | 0.370 | 0.230 | -0.141 |
| 7 | 0.586 | 0.560 | -0.026 |
| 8 | 0.245 | 0.253 | 0.008 |
| 9 | 0.286 | 0.183 | -0.103 |
| 10 | 0.445 | 0.390 | -0.055 |
| 11 | 0.224 | 0.281 | 0.057 |
| Mean | 0.354 | 0.318 | -0.036 |
| Min | 0.201 | 0.167 | -0.141 |
| Max | 0.586 | 0.560 | 0.057 |
| SD | 0.137 | 0.138 | 0.066 |
| Median | 0.328 | 0.267 | -0.030 |
| Δ% | | | -10% |
| % of volunteers with positive effect | | | 60% |

| | | | |
|---|---|---|---|
| Legend: ()* - Result not included in the calculation; AV - incorrect value | | | |

On the basis of conducted application studies, after 28 days of regular application, it was found that the extract prepared according to the invention at a concentration of 100 mg/L, makes the skin more elastic (on average by 6%) and firms the skin (on average by 10%).

### 12. Analysis of wrinkles using Antera 3D^{®}

The aim of the study was to confirm the wrinkle reducing activity on the depth and width of wrinkles in the measurement zone using Antera 3D^{®}. The study was conducted using a specialised measuring device from Miravex Limited. Eleven participants took part in the instrumental study. The study was conducted at the application site before (D0) and after 28 days (D28) of regular product application. The study was conducted in an air-conditioned room with a temperature of 20±2°C and a relative humidity of 50±10%. The activity of the extract is confirmed in the case of positive results obtained in more than 50% of the study participants. The extract reduces the depth (Table 12) and the width (Table 13) of wrinkles as the value of the parameters decreases.

**Table 12. Antera 3D^{®}. Results of wrinkle depth measurements measured before a start of using (D0) and after 28 days (D28) of regular product application measured in mm.**

| **Volunteer No.** | **Before (D0)** | **After 28 days (D28)** | **Difference (D28-D0)** |
|---|---|---|---|
| 1 | 1.7830 | 1.8510 | 0.068 |
| 2 | 1.9900 | 1.8850 | -0.105 |
| 3 | 1.9550 | 1.9210 | -0.034 |
| 4 | 1.9170 | 1.8380 | -0.079 |
| 5 | 1.8510 | 1.7910 | -0.060 |
| 6 | 1.9600 | 1.8940 | -0.066 |
| 7 | 1.6840 | 1.6250 | -0.059 |
| 8 | 1.8380 | 1.7630 | -0.075 |
| (9)* | (1.8250)* | AV | |
| 10 | 1.9660 | 1.7800 | -0.186 |
| 11 | 1.8510 | 1.7910 | -0.060 |
| Mean | 1.880 | 1.814 | -0.066 |
| Min | 1.684 | 1.625 | -0.186 |
| Max | 1.990 | 1.921 | 0.068 |
| SD | 0.097 | 0.085 | 0.063 |
| Median | 1.884 | 1.815 | -0.063 |
| Δ% | | | -3% |
| % of volunteers with positive effect | | | 90% |

| | | | |
|---|---|---|---|
| Legend: ()* - Result not included in the calculation; AV - incorrect value | | | |

**Table 13. Antera 3D^{®}. Results of wrinkle widths measurements measured before a start of using (D0) and after 28 days (D28) of regular product application measured in mm.**

| **Volunteer No.** | **Before (D0)** | **After 28 days (D28)** | **Difference (D28-D0)** |
|---|---|---|---|
| 1 | 0.485 | 0.524 | 0.039 |
| 2 | 0.475 | 0.366 | -0.110 |
| 3 | 0.201 | 0.167 | -0.034 |
| (4)* | (0.412)* | AV | |
| 5 | 0.223 | 0.230 | 0.007 |
| 6 | 0.370 | 0.230 | -0.141 |
| 7 | 0.586 | 0.560 | -0.026 |
| 8 | 0.245 | 0.253 | 0.008 |
| 9 | 0.286 | 0.183 | -0.103 |
| 10 | 0.445 | 0.390 | -0.055 |
| 11 | 0.224 | 0.281 | 0.057 |
| Mean | 0.354 | 0.318 | -0.036 |
| Min | 0.201 | 0.167 | -0.141 |
| Max | 0.586 | 0.560 | 0.057 |
| SD | 0.137 | 0.138 | 0.066 |
| Median | 0.328 | 0.267 | -0.030 |
| Δ% | | | -10% |
| % of volunteers with positive effect | | | 60% |

| | | | |
|---|---|---|---|
| Legend: ()* - Result not included in the calculation; AV - inappropriate value. | | | |

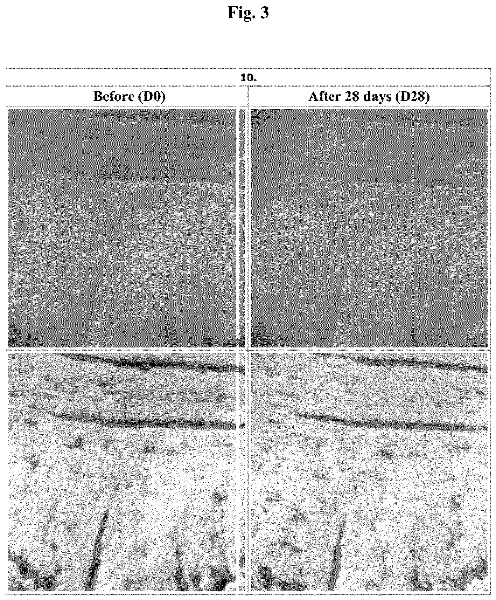

Visual representation of the results, showing the studied site before a start of using (D0) and after 28 days (D28) of regular product application (Fig. 3).

On the basis of the conducted application studies, after 28 days of regular application, it was found that the extract prepared according to the invention at a concentration of 100 mg/L, reduces the depth (by 6% on average) and the width of wrinkles (by 3% on average).

### Advantageous Effects of Invention

According to the invention, the developed solution extends the range of raw materials for obtaining cosmetic preparations and compositions as well as dietary supplements, comprising a plant extract of *Impatiens glandulifera* Royle with the addition of *Pandanus amaryllifolius* Roxb.

According to the invention, the extract shows many beneficial properties, relevant for anti-aging activity on the skin, through a very strong collagenase and elastase inhibitory activity, antioxidant activity due to the high polyphenol content, anti-inflammatory activity being a strong lipoxygenase inhibitor, skin brightening (whitening) activity due to inhibition of tyrosinase activity, and anti-wrinkle activity causing improved skin moisturizing and smoothness, as well as improvement of skin biomechanical parameters such as skin elasticity, skin firmness, and activity of reducing the depth and the width of wrinkles. It was also confirmed that the extract according to the invention has no cytotoxic effect against human skin fibroblasts (at concentrations of 1.95-250 µg of the extract per 1 mL of culture medium).

Furthermore, it was found that the extract according to the invention (100 mg/L of glycerol) used by people who have not been documented to be allergic to any one of the ingredients is well tolerated by the skin. No irritation or allergic reactions occurred in the studied group of volunteers. The extract according to the invention therefore meets the requirements of the Skin Compatibility Test and can be classified as non-irritating.

This extract can be added as an active ingredient to the cosmetic preparations, because it shows antioxidant, whitening (skin brightening) and anti-wrinkle effects confirmed by research.

## Claims

1. An extract of flowers, leaves and/or seed pods of Impatiens and leaves of Pandanus, in dry and/or liquid form, **characterized in that** it is obtained from *Impatiens glandulifera* with the addition of *Pandanus amaryllifolius,* wherein the extract has a total polyphenol content ranging from 13.0 to 51.0 mg of gallic acid equivalent per g of dry extract.

2. The extract according to claim 1, **characterized in that** it contains:
| No. | Compund | Content [mg/g of dry extract] |
|---|---|---|
| 1 | *p*-hydroxybenzoic acid | 18.32 - 20.05 |
| 2 | chlorogenic acid | 3.27 - 3.72 |
| 3 | azelaic acid | 20.89 - 21.33 |
| 4 | *p*-coumaric acid | 2.19 - 2.61 |
| 5 | ferulic acid | 3.52 - 3.93 |
| 6 | vanillic acid | 4.50 - 4.71 |
| 7 | salicylic acid | 2.54 - 2.83 |
| 8 | kaempferol 3-*O*-rutinoside | 2.38 - 2.61 |
| 9 | hyperoside | 1.73 - 1.90 |
| 10 | isoquercitrin | 2.65 - 2.88 |
| 11 | quercitrin | 2.26 - 2.59 |
| 12 | kaempferol 7-*O*-glucoside | 3.93 - 4.26 |
| 13 | astragalin | 7.24 - 7.81 |
| 14 | kaempferol | 11.99 - 13.08 |

3. The extract according to claim 1 or 2, **characterized in that** it is in the form of a dry extract as a lyophilisate.

4. The extract according to any one of claims 1 to 3, **characterized in that** it is in a liquid form which is a glycerol solution, preferably in the proportion of 1 part by weight of dry extract and 100-250 parts by weight of glycerol.

5. A method of obtaining an extract of *Impatiens glandulifera* with the addition of *Pandanus amaryllifolius* as defined in claim 1, **characterized in that** it comprises the following steps:
(a). providing a material in the form of 9.5-12.0 parts by weight of flowers, leaves and/or seed pods of *Impatiens glandulifera* and 0.5-1.0 part by weight of leaves of *Pandanus amaryllifolius;*
(b). subjecting the material from (a) to ultrasonic extraction in a bath at the power of 360 W, the frequency of 45 kHz, in 55-100 parts by weight of a 60-90% aqueous ethanol solution and at a temperature of 20-70°C, for 15-90 minutes, producing the aqueous-ethanolic fraction of the extract;
(c). subjecting the aqueous-ethanolic fraction of the extract obtained in (b) to filtration;
(d). concentration of the product obtained in (c), lyophilisation and storage below room temperature thereof, with obtaining a dry extract form as a lyophilisate, optionally
(e). dissolving the dry extract obtained in (d) in vegetable glycerol in the proportion of 1 part by weight of dry extract and 500-1250 parts by weight of vegetable glycerol to produce a liquid form of this extract.

6. The method according to claim 5, **characterized in that** in (a) a material comprising 3 parts by weight of flowers, 6-8 parts by weight of leaves, 0.5-1.0 part by weight of seed pods of *Impatiens glandulifera* and 0.5-1.0 part by weight of leaves of *Pandanus amaryllifolius* is used.

7. The method according to claim 5 or 6, **characterized in that** the extraction in (b) is carried out in 60-90 parts by weight of a 70-80% aqueous ethanol solution.

8. The method according to any one of claims 5 to 7, **characterized in that** the extraction in (b) is carried out at 40-50°C.

9. The method according to any one of claims 5 to 8, **characterized in that** the extraction in (b) is carried out for 30-40 minutes.

10. The method according to any one of claims 5 to 9, **characterized in that** the lyophilised extract from (d) is stored at a temperature below 10°C.

11. The method according to any one of claims 5 to 10, **characterized in that** in (e) 900-1150 parts by weight of vegetable glycerol are used per 1 part by weight of dry extract.

12. The plant extract of *Impatiens glandulifera* with the addition of *Pandanus amaryllifolius,* as defined in claim 1, **characterized in that** it is obtained by the method according to claim 5.

13. An non-therapeutic use of the extract of *Impatiens glandulifera* and *Pandanus amaryllifolius,* as defined in claim 1 or 12, as an agent having anti-aging, antioxidant, skin-brightening, anti-wrinkle properties and inhibiting the activity of collagenase, elastase and tyrosinase, either in cosmetics or in dietary supplements.

14. The use according to claim 13, **characterized in that** the extract is present in anti-wrinkle creams, preferably in the amount of 4% by weight per 100% by weight of the total composition.

## Patentansprüche

1. Extrakt aus Blüten, Blättern und/oder Samenschoten vom Springkraut und Pandanblättern, in trockener und/oder flüssiger Form, **dadurch gekennzeichnet, dass** er aus *Impatiens glandulifera* unter Zugabe von *Pandanus amaryllifolius* gewonnen wird, wobei der Gesamtgehalt an Polyphenolen im Extrakt zwischen 13,0 und 51,0 mg Gallussäure-Äquivalent pro Gramm Trockenextrakt beträgt.

2. Extrakt nach Anspruch 1, **dadurch gekennzeichnet, dass** er enthält:
| Pos. | Verbindung | Gehalt [mg/g Trockenextrakt] |
|---|---|---|
| 1 | *p*-Hydroxybenzoesäure | 18,32 - 20,05 |
| 2 | Chlorogensäure | 3,27 - 3,72 |
| 3 | Azelainsäure | 20,89 - 21,33 |
| 4 | *p*-Kumarsäure | 2,19 - 2,61 |
| 5 | Ferulasäure | 3,52 - 3,93 |
| 6 | Vanillinsäure | 4,50 - 4,71 |
| 7 | Salicylsäure | 2,54 - 2,83 |
| 8 | 3-O-Rutinosid von Kaempferol | 2,38 - 2,61 |
| 9 | Hyperosid | 1,73 - 1,90 |
| 10 | Isoquercitrin | 2,65 - 2,88 |
| 11 | Quercitrin | 2,26 - 2,59 |
| 12 | 7-O-Glucosid von Kaempferol | 3,93 - 4,26 |
| 13 | Astragalin | 7,24 - 7,81 |
| 14 | Kaempferol | 11,99 - 13,08 |

3. Extrakt nach Anspruch 1 oder 2, **dadurch gekennzeichnet, dass** es sich um einen Trockenextrakt in Form eines Lyophilisats handelt.

4. Extrakt nach einem der Ansprüche 1 bis 3, **dadurch gekennzeichnet, dass** es sich um eine flüssige Form handelt, die eine Glycerinlösung ist, vorzugsweise im Verhältnis von 1 Gewichtsteil des Trockenextrakts zu 100 bis 250 Gewichtsteilen Glycerin.

5. Verfahren zur Gewinnung eines Extrakts aus *Impatiens glandulifera* mit Zusatz von *Pandanus amaryllifolius,* wie in Anspruch 1 angegeben, **dadurch gekennzeichnet, dass** es die folgenden Schritte umfasst:
(a). Bereitstellen des Materials in Form von 9,5-12,0 Gewichtsteilen Blüten, Blättern und/oder Samenschoten von *Impatiens glandulifera* und 0,5-1,0 Gewichtsteilen Blättern von *Pandanus amaryllifolius;*
(b). Durchführen einer Ultraschallextraktion des Materials aus (a) in einem Ultraschallbad bei einer Leistung von 360 W und einer Frequenz von 45 kHz in 55-100 Gewichtsteilen einer 60-90%-igen wässrigen Ethanollösung bei einer Temperatur von 20 -70 °C für 15-90 Minuten, wodurch eine Wasser-Ethanol-Fraktion des Extrakts gewonnen wird;
(c) Filtrieren der in (b) erhaltenen Wasser-Ethanol-Fraktion des Extrakts;
(d). Eindicken des in (c) erhaltenen Produkts, Gefriertrocknen und Lagern bei einer Temperatur unterhalb der Raumtemperatur, wodurch die Trockenform des Extrakts als Lyophilisat erhalten wird, wahlweise
(e). Auflösen des in (d) erhaltenen Trockenextrakts in pflanzlichem Glycerin im Verhältnis von 1 Gewichtsteil Trockenextrakt zu 500-1250 Gewichtsteilen pflanzlichem Glycerin, zur Herstellung einer flüssigen Form dieses Extrakts.

6. Verfahren nach Anspruch 5, **dadurch gekennzeichnet, dass** in (a) ein Material verwendet wird, das 3 Gewichtsteile Blüten, 6-8 Gewichtsteile Blätter, 0,5-1,0 Gewichtsteile Samenschoten von *Impatiens glandulifera* sowie 0,5-1,0 Gewichtsteile Blätter von *Pandanus amaryllifolius* enthält.

7. Verfahren nach Anspruch 5 oder 6, **dadurch gekennzeichnet, dass** die Extraktion in (b) in 60-90 Gewichtsteilen einer 70-80 %-igen wässrigen Ethanol-Lösung durchgeführt wird.

8. Verfahren nach einem der Ansprüche 5 bis 7, **dadurch gekennzeichnet, dass** die Extraktion in (b) bei einer Temperatur von 40 bis 50 °C durchgeführt wird.

9. Verfahren nach einem der Ansprüche 5 bis 8, **dadurch gekennzeichnet, dass** die Extraktion in (b) über einen Zeitraum von 30 bis 40 Minuten durchgeführt wird.

10. Verfahren nach einem der Ansprüche 5 bis 9, **dadurch gekennzeichnet, dass** der gefriergetrocknete Extrakt aus (d) bei einer Temperatur unter 10 °C gelagert wird.

11. Verfahren nach einem der Ansprüche 5 bis 10, **dadurch gekennzeichnet, dass** in (e) 900 bis 1150 Gewichtsteile pflanzliches Glycerin pro 1 Gewichtsteil Trockenextrakt verwendet werden.

12. Pflanzenextrakt aus *Impatiens glandulifera* mit Zusatz von *Pandanus amaryllifolius,* wie in Anspruch 1 angegeben, **dadurch gekennzeichnet, dass** er nach dem Verfahren gemäß Anspruch 5 gewonnen wird.

13. Nichttherapeutische Verwendung des Extrakts aus *Impatiens glandulifera* und *Pandanus amaryllifolius,* wie in Anspruch 1 oder 12 angegeben, als Mittel mit Anti-Aging-, antioxidativen, hautaufhellenden und faltenmindernden Eigenschaften sowie zur Hemmung der Aktivität von Kollagenase, Elastase und Tyrosinase, sowohl in Kosmetika als auch in Nahrungsergänzungsmitteln.

14. Verwendung nach Anspruch 13, **dadurch gekennzeichnet, dass** der Extrakt in Anti-Falten-Cremes enthalten ist, vorzugsweise in einer Menge von 4 Gew.-% pro 100 Gew.-% der Gesamtzusammensetzung.

## Revendications

1. Extrait de fleurs, de feuilles et/ou de gousses d'impatiente et de de feuilles pandanus, sous forme sèche et/ou liquide, **caractérisé en ce qu'il** est obtenu à partir d*'Impatiens glandulifera* avec l'ajout de *Pandanus amaryllifolius,* la teneur totale en polyphénols de l'extrait étant comprise entre 13,0 et 51,0 mg d'équivalent d'acide gallique par gramme d'extrait sec.

2. Extrait selon la revendication 1, **caractérisé en ce qu'il** contient :
| N° | Composé | Teneur [mg/g d'extrait sec] |
|---|---|---|
| 1 | acide *p*-hydroxybenzoïque | 18,32 - 20,05 |
| 2 | acide chlorogénique | 3,27 - 3,72 |
| 3 | acide azélaïque | 20,89 - 21,33 |
| 4 | acide *p*-coumarique | 2,19 - 2,61 |
| 5 | acide férulique | 3,52 - 3,93 |
| 6 | acide vanillique | 4,50 - 4,71 |
| 7 | acide salicylique | 2,54 - 2,83 |
| 8 | 3-*O*-rutinoside du kaempférol | 2,38 - 2,61 |
| 9 | hyperoside | 1,73 - 1,90 |
| 10 | isoquercitrine | 2,65 - 2,88 |
| 11 | quercitrine | 2,26 - 2,59 |
| 12 | 7-*O*-glucoside du kaempférol | 3,93 - 4,26 |
| 13 | astragaline | 7,24 - 7,81 |
| 14 | kaempférol | 11,99 - 13,08 |

3. Extrait selon la revendication 1 ou 2, **caractérisé en ce qu'il** se présente sous la forme d'un extrait sec lyophilisé..

4. Extrait selon l'une quelconque des revendications 1 à 3, **caractérisé en ce qu'il** se présente sous forme liquide qui est une solution de glycérol, avantageusement dans un rapport de 1 partie en poids d'extrait sec pour 100 à 250 partes en poids de glycérol.

5. Procédé d'obtention de l'extrait d*'Impatiens glandulifera* avec ajout de *Pandanus amaryllifolius,* tel que défini dans la revendication 1, **caractérisé en ce qu'il** comprend les étapes suivantes :
(a). préparation du matériau composé de 9,5 à 12,0 parties en poids de fleurs, de feuilles et/ou de gousses d*'Impatiens glandulifera* et de 0,5 à 1,0 partie en poids de feuilles de *Pandanus amaryllifolius ;*
(b). extraction du matériau visé au point (a) par ultrasons d'une puissance de 360 W et d'une fréquence de 45 kHz, dans un bain avec 55 à 100 parties en poids d'une solution aqueuse d'éthanol à 60-90 %, à une température de 20 à -70 °C, pendant 15 à 90 minutes, ce qui donne une fraction hydro-éthanolique de l'extrait ;
(c). filtration de la fraction hydro-éthanolique de l'extrait obtenue au point (b) ;
(d). concentration du produit obtenu au point (c), lyophilisation et conservation à une température inférieure à la température ambiante, pour obtenir la forme sèche de l'extrait sous forme de lyophilisat, en option
(e). dissolution de l'extrait sec obtenu au point (d) dans du glycérol végétal dans un rapport de 1 partie en poids d'extrait sec pour 500 à 1 250 parties en poids de glycérol végétal, afin d'obtenir une forme liquide de cet extrait.

6. Procédé selon la revendication 5, **caractérisé en ce que,** au point (a), on utilise un matériau contenant 3 parties en poids de fleurs, 6 à 8 parties en poids de feuilles, 0,5 à 1,0 partie en poids de gousses *d'Impatiens glandulifera* et 0,5 à 1,0 patrie en poids de feuilles de *Pandanus amaryllifolius.*

7. Procédé selon la revendication 5 ou 6, **caractérisé en ce que** l'extraction au point (b) est effectuée dans 60 à 90 parties en poids d'une solution aqueuse d'éthanol à 70-80 %.

8. Procédé selon l'une quelconque des revendications 5 à 7, **caractérisé en ce que** l'extraction au point (b) est effectuée à une température de 40 à 50°C.

9. Procédé selon l'une quelconque des revendications 5 à 8, **caractérisé en ce que** l'extraction au point (b) est effectuée pendant 30 à 40 minutes.

10. Procédé selon l'une quelconque des revendications 5 à 9, **caractérisé en ce que** l'extrait lyophilisé du (d) est conservé à une température inférieure à 10°C.

11. Procédé selon l'une quelconque des revendications 5 à 10, **caractérisé en ce que,** au point (e), on utilise 900 à 1150 parties en poids de glycérol végétal pour 1 parte en poids d'extrait sec.

12. Extrait végétal *d'Impatiens glandulifera* avec ajout de *Pandanus amaryllifolius,* tel que défini dans la revendication 1, **caractérisé en ce qu'il** est obtenu par le procédé selon la revendication 5.

13. Utilisation non thérapeutique de l'extrait *d'Impatiens glandulifera* et de *Pandanus amaryllifolius,* tel que défini dans la revendication 1 ou 12, en tant qu'agent aux propriétés antivieillissement, antioxydantes, éclaircissantes de la peau, antirides et inhibant l'activité de la collagénase, de l'élastase et de la tyrosinase, tant dans les cosmétiques que dans les compléments alimentaires.

14. Utilisation selon la revendication 13, **caractérisée en ce que** l'extrait est présent dans des crèmes antirides, avantageusement à raison de 4 % en poids pour 100 % en poids de la composition totale.
